# EUROPEAN PATENT APPLICATION

(11) **EP 4 706 739 A1**
(43) Date of publication of application: **11.03.2026**
(21) Application number: 24198579.5
(22) Date of filing: 05.09.2024
(51) Int. Cl.: A61M 39/24, A61J 15/00

(54) **ENTERAL GRAVITY-FEEDING SYSTEM AND ANTI-REFLUX VALVE FOR SUCH SYSTEM**

(71) Applicant: Umbilicare, 7500 Holstebro (DK)
(72) Inventor: Schultz, Michael, 7500 Holstebro (DK)
(74) Representative: Larsen & Birkeholm A/S

(57) **Abstract**

The present invention relates to an enteral gravity-feeding system, where the venting mechanism is integrated into the anti-reflux valve.

## Description

### Technical field of the invention

The present invention relates to the field of enteral gravity-feeding systems.

### Background of the invention

In neonatal intensive care units, many hospitalized babies, particularly premature infants, require feeding via a nasogastric or orogastric tube. This method is essential for providing nutrition to babies who cannot yet feed orally due to underdeveloped sucking and swallowing reflexes. However, one significant issue with tube feeding is the accumulation of gases in the stomach. These gases can cause discomfort, distension, and sometimes pain, which can lead to complications if not managed properly.

In many cases, the build-up of gas needs to be manually relieved by healthcare personnel or parents by aspirating the stomach contents through the feeding tube, a process that is both time-consuming and potentially distressing for the infant. This procedure is often referred to as "venting" or "decompressing" the stomach. Typically, this process may be performed by manual inspiration, intermittent venting, or continuous suction. Manual aspiration is performed when a healthcare provider or trained parent uses a syringe attached to the end of the feeding tube to gently pull back on the plunger, thereby creating suction. This suction helps to draw out any air, gas, and sometimes excess stomach contents that have accumulated in the baby's stomach. This method is straightforward and widely used, but is time-consuming and requires frequent monitoring, which increases the workload for healthcare providers and can be stressful for both the baby and the parents. In some cases, the feeding tube may be intermittently opened to air, allowing gas to escape passively. This is done by disconnecting the feeding syringe and leaving the tube open to the air for a short period. This technique relies on the natural release of gas from the stomach through the open tube. However, it is not always effective, especially if the gas is trapped within the stomach contents. In more severe cases, continuous low-pressure suction may be applied to the feeding tube using specialized equipment. This method is typically reserved for infants with significant gastrointestinal issues or those recovering from surgery. Continuous suction is more effective in managing gas but can also lead to other complications, such as irritation of the stomach lining or excessive removal of stomach contents, which can disrupt the baby's electrolyte balance and overall nutrition.

### Object of the Invention

The objective of the present invention is to provide a solution that can manage the accumulation of gas in a less invasive and more consistent manner to comply with the Newborn Individualized Developmental Care and Assessment Program (NIDCAP). NIDCAP emphasizes the importance of a supportive and nurturing environment for neonatal care. NIDCAP promotes minimizing stress and handling of the infant, aiming to create a more developmentally appropriate environment. Therefore, an ideal solution would automatically relieve the baby's stomach of gas without requiring frequent manual intervention. This would allow healthcare providers and parents to focus more on providing nurturing care rather than being preoccupied with mechanical procedures, thereby enhancing the overall well-being and development of the infant during this critical period.

### Summary of the Invention

A first aspect relates to an enteral gravity-feeding system comprising:
- a feeding container;
- a feeding set;
- a feeding tube;
- an anti-reflux valve; and
- a venting mechanism;
wherein the venting mechanism is integrated, at least partly, into the anti-reflux valve.

A second aspect relates to an anti-reflux valve comprising an integral venting mechanism.

In one or more embodiments, the venting mechanism is configured to allow controlled delivery of gas entering the anti-reflux valve, during use, into the feeding set.

In one or more embodiments, the anti-reflux valve comprises:
- a valve body with a central cavity and one or more peripheral channels or recesses;
- an inlet in fluid communication with the central cavity;
- an outlet in fluid communication with the central cavity; and
- a ball positioned within the central cavity;
wherein the one or more peripheral channels or recesses runs from a position below the inlet to a position above the outlet.

In one or more embodiments, the ball has a density of at most 0.8 grams per cubic centimetres (g/cm³), preferably within the range of 0.01-0.7 g/cm³, such as at most 0.7 grams per cubic centimetres (g/cm³), preferably within the range of 0.02-0.6 g/cm³, e.g., at most 0.6 grams per cubic centimetres (g/cm³), preferably within the range of 0.03-0.5 g/cm³, e.g., at most 0.4 grams per cubic centimetres (g/cm³), preferably within the range of 0.04-0.4 g/cm³, such as at most 0.3 grams per cubic centimetres (g/cm³), preferably within the range of 0.05-0.3 g/cm³, e.g., at most 0.2 grams per cubic centimetres (g/cm³), preferably within the range of 0.06-0.2 g/cm³, such as at most 0.1 grams per cubic centimetres (g/cm³), preferably within the range of 0.07-0.1 g/cm³, more preferably within the range of 0.01-0.1 g/cm³.

In one or more embodiments, the valve body includes one or more peripheral recesses that are integrated into inner walls defining the central cavity.

In one or more embodiments, the anti-reflux valve further comprises a gasket arranged at the top of the valve between the ball and the inlet.

In one or more embodiments, the central cavity comprises a first section, and a second section; wherein the first section is arranged at the top of the valve, and the second section is arranged below the first section; wherein the first section is relatively wider than the second section; and wherein the gasket is adapted to only move within the first section.

In one or more embodiments, the gasket is relatively wider than the ball.

As used in the specification and the appended claims, the singular forms "a", "an", and "the" include plural referents unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about", it will be understood that the particular value forms another embodiment.

It should be noted that embodiments and features described in the context of one of the aspects of the present invention also apply to the other aspects of the invention.

### Brief description of the figures

Figure 1A is a side view of an anti-reflux valve in accordance with various embodiments of the invention.
Figure 1B is a side view of an anti-reflux valve in accordance with various embodiments of the invention, where the walls are shown transparent.
Figure 2 is a perspective and exploded view of an anti-reflux valve in accordance with various embodiments of the invention.
Figure 3 is a cross-sectional view of an anti-reflux valve in accordance with various embodiments of the invention, where the ball is blocking the inlet.

### References

- 100: Anti-reflux valve
- 110: Valve body
- 112: Cavity
- 114: Recess
- 116: Inlet
- 118: Outlet
- 120: Ball
- 130: Gasket

### Detailed Description of the Invention

In the present context, the term "in general" when used when mentioning a feature relating to the present invention, it must be understood that the feature may be used with all embodiments of the invention, even if the mentioning is made in the detailed part of the document.

In general, enteral gravity-feeding systems are widely used for delivering nutrition to infants who require tube feeding. In these systems, the baby food or formula is placed in a container that is elevated above the level of the infant, allowing gravity to pull the food down through the feeding tube and into the baby's stomach.

The feeding container may be hung or placed on an adjustable pole or stand, typically at a height above the infant. The height can be adjusted to control the rate of flow, where raising the container increases the flow rate, while lowering it decreases the flow rate. The rate can also be adjusted using a clamp or roller on the tubing. By partially closing the clamp, the caregiver can slow down the flow, which is useful for infants who need to be fed more slowly to avoid overfeeding or reflux. The feeding container, also known or configured as a reservoir, syringe, or bag, holds the enteral nutrition formula or breast milk. It can be a soft, collapsible bag or a rigid bottle or syringe, depending on the specific system design. The container typically has graduated markings to allow caregivers to measure the precise amount of food being administered. It may also have a vent to allow air to enter as the liquid exits, preventing the formation of negative pressure.

In general, the feeding set is a length of medical-grade tubing that connects the feeding container to the feeding tube. The feeding set may include various parts, such as a drip chamber, clamps, and connectors. Located near the top of the feeding set, the drip chamber may help control the flow of the liquid and allows caregivers to monitor the rate of flow visually. A roller or slide clamp may be included to regulate or stop the flow of nutrition from the container to the feeding tube. The feeding set typically ends in a connector that attaches securely to the feeding tube, ensuring a leak-proof and sterile connection.

In general, the feeding tube is the component that delivers the nutrition directly into the patient's stomach or small intestine. Feeding tubes can be nasogastric (NG), orogastric (OG), or gastrostomy tubes (G-tubes), depending on how they are inserted and the patient's needs. It is typically made from soft, flexible materials, like silicone or polyurethane, to minimize discomfort and reduce the risk of injury.

In general, and only in some systems, particularly those designed for infants, a venting mechanism is included to allow trapped gas to escape from the stomach, thereby reducing discomfort and preventing gastric distension.

Typically, the venting is managed manually through syringe aspiration or automatically through specialized vented tubing or a venting port integrated into the tubing.

In general, the anti-reflux valve is a one-way valve that prevents the backflow of stomach contents into the feeding tube. This valve is critical for reducing the risk of aspiration and ensuring that the feeding process is unidirectional, i.e., from the container to the stomach. The valve can be a separate component or integrated into the feeding set or tube, depending on the system.

In prior art systems, the valve itself often comprises a small flap or diaphragm that moves in response to pressure changes, allowing for fluid movement in one direction while blocking it in the other.

The core of the present invention is to integrate the venting mechanism into the anti-reflux valve. The advantage of this approach is to secure venting during feeding. This embodiment may e.g., be realized by constructing the anti-reflux valve as a specially designed one-way ball valve.

In general, the anti-reflux one-way ball valve comprises a valve body with a central cavity and one or more peripheral channels or recesses, an inlet in fluid communication with the central cavity, an outlet in fluid communication with the central cavity, and a ball positioned within the central cavity.

The valve body is preferably designed for vertical installation, and with an inlet at the top and an outlet at the bottom. The internal diameter of the valve body accommodates the ball, allowing it to move freely up and down in response to pressure changes.

The ball is the key component, made from a material with a density lower than water/the liquid food, such as a hollow plastic or composite material. The ball is (slightly) smaller than the internal diameter of the valve central cavity.

The inner walls of the valve body that defines the central cavity include channels or grooves that allow liquid to bypass the ball when it is not seated/pushed towards the inlet, i.e., even when the ball is seated at the bottom of the cavity. These channels ensure that liquid can flow unobstructed through the valve during feeding.

In the context of enteral feeding, particularly in neonatal care, the term "reflux" refers to the backward flow of stomach contents into the oesophagus, back into the feeding system. This backward flow occurs when the contents of the stomach, including gastric acid, partially digested food, or enteral nutrition, move up the gastrointestinal tract rather than continuing down into the intestines as intended. This can occur if the pressure in the stomach exceeds the pressure in the feeding tube. Another situation where pressure builds up in the stomach is due to gasses. When gases build up in the stomach of neonates, it is commonly referred to as gastric distension or aerophagia. Gastric distension specifically describes the condition where the stomach becomes enlarged or swollen due to the accumulation of gas. In neonates, this can happen because they often swallow air during feeding or crying, or because of fermentation of food in the stomach. Gastric distension can lead to discomfort, bloating, and in some cases, it may cause reflux as the increased pressure in the stomach pushes contents upward. Aerophagia refers to the swallowing of air, which can lead to the accumulation of gas in the stomach. In neonates, aerophagia is common due to immature feeding techniques, the use of feeding tubes, or crying, which all contribute to the intake of air. All these situations result in a pressure working against the flow direction of the food.

The space below the ball within the central cavity allows for gas buildup. This space is important for the operation of the venting mechanism, as it allows gas to accumulate. When the pressure drops again, the ball will drop a little, and the accumulated gas will be able to escape through the valve inlet and into the tubing. Eventually, this gas will end up in the feeding container, where it may be vented into the atmosphere or simply be used to substitute the food within the container.

Hence, under normal conditions, liquid food enters the valve through the inlet at the top. The ball, which is less dense than the liquid, may be seated within the central cavity or float on the liquid, and thus does not block the outlet, allowing liquid food to flow around it through the peripheral channels or recesses and exit through the outlet at the bottom.

A gasket may be positioned between the ball and the inlet at the top. The preferably disc-shaped gasket ensures a tight seal when the ball is pressed upward. This gasket may be designed to deform slightly to conform to the shape of the ball, creating an effective seal that prevents liquid or gas from passing through.

The gasket remains unengaged during normal flow, and the liquid flows smoothly through the valve.

If gas accumulates below the ball, the pressure increases within the gas relief space. This pressure pushes the ball upward, pressing it against the inlet. The gasket enhances the seal, e.g., by conforming to the shape of the ball, preventing any liquid or gas from passing through the valve. As the gas pressure decreases, the ball lowers due to gravity, disengaging from the gasket and opening the inlet. The gas can then escape through the inlet, and the valve returns to its open state, allowing liquid to flow through the peripheral channels or recesses and out of the valve.

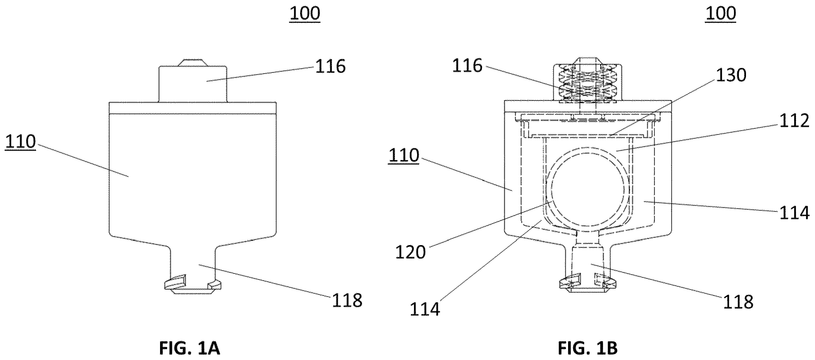

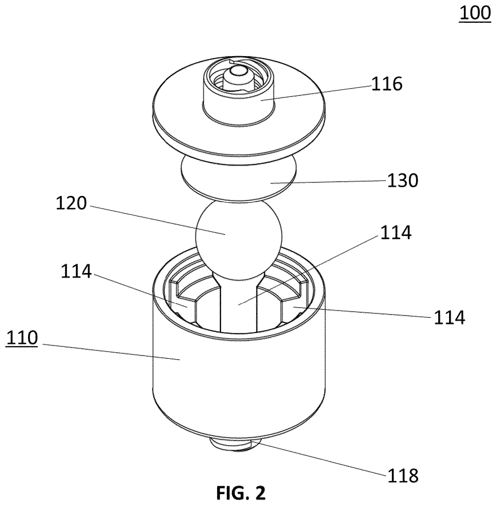

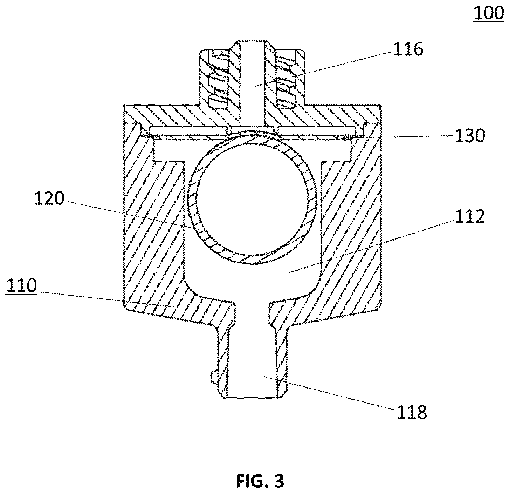

Figures 1-3 illustrate preferred embodiments of an anti-reflux valve according to the invention.

Figure 1A shows a side view of anti-reflux valve 100 according to the invention, and Figure 1B shows the same view, where the walls are made transparent for a better view of the individual components. The anti-reflux valve 100 comprises a valve body 110 with a central cavity 112 and a plurality (here four, only three of those can be seen in the figures) of peripheral recesses 114, an inlet 116 in fluid communication with the central cavity, and an outlet 118 in fluid communication with the central cavity 112. A ball 120 is positioned within the central cavity 112. In general, the peripheral recesses 114 may run from a position below the inlet 116 to a position above the outlet 118.

In general, multiple recesses 114 distributed around the periphery of the cavity 112 can help create a more even and balanced flow around the ball 120. This can reduce turbulence and ensure that fluid passes smoothly through the valve 100, even if the ball 120 is partially obstructing the central flow path. With multiple recesses 114, there is an inherent redundancy in the flow paths. If one or more recesses become partially obstructed (e.g., by debris), the other recesses can still allow fluid to pass, maintaining the valve's functionality. A single recess is generally also possible and can potentially allow for a greater volume of fluid to flow through the valve at once. However, a single wide recess may create more turbulence within the cavity 112.

In the disclosed embodiment, the central cavity 112 of the valve is designed with a wider upper section near the inlet to house a disc-shaped gasket 130 with a diameter larger than the ball 120, allowing the gasket 130 to move freely back and forth relative to the inlet 116. The lower section of the cavity 112 is narrower, precisely shaped to hold the ball 120, which can move up and down to open or close the valve, and without excessive lateral movement. This configuration ensures that the gasket 130 can seal effectively without obstructing the ball's movement, maintaining the valve's functionality.

In general, for a ball valve application, the material chosen for the ball 120 must not only be lightweight but also capable of withstanding mechanical stress, including pressure from liquid or gas, friction against the valve body, and potential impacts during operation.

Expanded Polypropylene (EPP) has a density of within the range of 0.02 to 0.06 g/cm³. EPP is a strong, flexible, and durable material that is known for its impact resistance and ability to withstand repeated mechanical stress without deforming. It's also resistant to a wide range of chemicals.

Microspheres or polymer-based hollow spheres generally have a density of within the range of 0.02 to 0.1 g/cm³. Polymer-based microspheres embedded within a polymer matrix can be used to create a composite material that retains low density while offering enhanced mechanical properties. While the microspheres themselves might not be strong enough alone, when used within a composite material, they can help create a lightweight but robust ball that can withstand mechanical stresses in a valve. This approach allows for the creation of a custom material that balances low density with necessary strength and durability.

Using a ball with a central cavity is generally an alternative to using materials like expanded polypropylene (EPP) or microspheres. A ball with a central cavity can offer several benefits, including reduced overall density and the ability to fine-tune the ball's buoyancy and mechanical properties. The ball would consist of a solid outer shell made from a strong, lightweight material, such as polypropylene (PP), polyethylene (PE), or another suitable polymer. This shell would provide the necessary mechanical strength to withstand the stresses encountered in the valve. Inside the ball, a central cavity would be incorporated, which significantly reduces the overall mass of the ball. This cavity could be empty (air-filled) or filled with a very low-density material (like foam or gas) to achieve the desired buoyancy. By creating a hollow cavity inside the ball, the overall density of the ball can be reduced, making it comparable to or even lower than other lightweight materials, like EPP or microspheres. This would allow the ball to float effectively and respond to fluid and gas pressures within the valve. The size and shape of the central cavity can be adjusted to fine-tune the ball's buoyancy. For example, making the cavity larger would reduce the ball's density further, allowing for precise control over how the ball interacts with the fluid in the valve. Using a central cavity design allows the outer shell to be made from a variety of materials, depending on the specific needs of the application (e.g., chemical resistance, temperature tolerance). A ball with a central cavity can be manufactured using techniques like blow moulding or injection moulding, where the cavity is formed during the moulding process. These techniques are well-established and can produce high-quality, consistent products.

The disclosed valve 100 is equipped with ENFit connectors at both the inlet and outlet, ensuring secure and standardized (ISO 80369-3) connections to the feeding set, and feeding tube, respectively. In general, these connectors are specifically designed for enteral feeding applications, ensuring compatibility with a wide range of ENFit-compliant tubing and devices. The ENFit connectors feature a locking mechanism that ensures a secure and leak-proof connection. The threaded design prevents accidental disconnections and ensures that the connections remain secure during operation. ENFit connectors are designed to prevent misconnections with non-enteral devices, enhancing patient safety by reducing the risk of cross-connection with incompatible systems.

## Claims

1. An enteral gravity-feeding system comprising:
- a feeding container;
- a feeding set;
- a feeding tube;
- an anti-reflux valve; and
- a venting mechanism;
**characterized in that** the venting mechanism is integrated into the anti-reflux valve.

2. The enteral gravity-feeding system according to claim 1, wherein the venting mechanism is configured to allow controlled delivery of gas entering the anti-reflux valve, during use, into the feeding set.

3. The enteral gravity-feeding system according to any one of the claims 1-2, wherein the anti-reflux valve comprises:
- a valve body with a central cavity and one or more peripheral channels or recesses;
- an inlet in fluid communication with the central cavity;
- an outlet in fluid communication with the central cavity; and
- a ball positioned within the central cavity;
wherein the one or more peripheral channels or recesses runs from a position below the inlet to a position above the outlet.

4. The enteral gravity-feeding system according to claim 3, wherein the ball has a density of at most 0.8 grams per cubic centimetres (g/cm³), preferably within the range of 0.02-0.7 g/cm³.

5. The enteral gravity-feeding system according to any one of the claims 3-4, wherein the valve body includes one or more peripheral recesses that are integrated into inner walls defining the central cavity.

6. The enteral gravity-feeding system according to any one of the claims 3-5, wherein the anti-reflux valve further comprises a gasket arranged at the top of the valve between the ball and the inlet.

7. The enteral gravity-feeding system according to claim 6, wherein the central cavity comprises a first section, and a second section; wherein the first section is arranged at the top of the valve, and the second section is arranged below the first section; wherein the first section is relatively wider than the second section; and wherein the gasket is adapted to only move within the first section.

8. The enteral gravity-feeding system according to any one of the claims 6-7, wherein the gasket is relatively wider than the ball.

9. An anti-reflux valve for use with an enteral gravity-feeding system, the anti-reflux valve comprising an integral venting mechanism.

10. The anti-reflux valve according to claim 9, wherein the anti-reflux valve comprises:
- a valve body with a central cavity and one or more peripheral channels or recesses;
- an inlet in fluid communication with the central cavity;
- an outlet in fluid communication with the central cavity; and
- a ball positioned within the central cavity;
wherein the one or more peripheral channels or recesses runs from a position below the inlet to a position above the outlet.

11. The anti-reflux valve according to claim 10, wherein the ball has a density of at most 0.8 grams per cubic centimetres (g/cm³), preferably within the range of 0.02-0.7 g/cm³.

12. The anti-reflux valve according to any one of the claims 10-11, wherein the valve body includes one or more peripheral recesses that are integrated into inner walls defining the central cavity.

13. The anti-reflux valve according to any one of the claims 10-12, further comprising a gasket arranged at the top of the valve between the ball and the inlet.

14. The anti-reflux valve according to claim 13, wherein the central cavity comprises a first section, and a second section; wherein the first section is arranged at the top of the valve, and the second section is arranged below the first section; wherein the first section is relatively wider than the second section; and wherein the gasket is adapted to only move within the first section.

15. The anti-reflux valve according to any one of the claims 13-14, wherein the gasket is relatively wider than the ball.
